## Europäisches Patentamt
## European Patent Office
## Office européen des brevets

(19)

(11) Numéro de publication: **0 146 464**
**B1**

(12) # FASCICULE DE BREVET EUROPÉEN

(45) Date de publication du fascicule du brevet:
**05.11.86**

(51) Int. Cl.⁴: **C 07 C 49/167, C 07 C 45/63**

(21) Numéro de dépôt: **84402517.1**

(22) Date de dépôt: **06.12.84**

(54) **Procédé catalytique de préparation d'hexafluoroacétone.**

(30) Priorité: **13.12.83 FR 8319915**

(43) Date de publication de la demande:
**26.06.85 Bulletin 85/26**

(45) Mention de la délivrance du brevet:
**05.11.86 Bulletin 86/45**

(84) Etats contractants désignés:
**DE FR GB IT NL**

(56) Documents cité:
**DE-A-1 443 616**
**FR-A-1 452 159**
**FR-A-2 034 585**

**Le dossier contient des informations techniques
présentées postérieurement au dépôt de la
demande et ne figurant pas dans le présent
fascicule.**

(73) Titulaire: **ATOCHEM, 4 & 8, Cours Michelet La
Défense 10, F-92800 Puteaux (FR)**

(72) Inventeur: **Cheminal, Bernard Résidence
Beauséjour, Saint- Irénée 26, rue Soeur Bouvier,
F-69005 Lyon (FR)**
Inventeur: **Mathias, Henri Hameau de Saint-
Didier, Villa No. 2 Chemin de l'Indiennerie,
F-69370 Saint- Didier au Mont d'Or (FR)**

(74) Mandataire: **Leboulenger, Jean, ATOCHEM
Département Propriété Industrielle, F-92091 Paris
la Défense 10 Cédex 42 (FR)**

LIBER, STOCKHOLM 1986

EP 0 146 464 B1

## Description

La présente invention concerne un procédé catalytique continu de préparation d'hexafluoroacétone par fluoruration de l'hexachloroacétone en phase gazeuse par l'acide fluorhydrique anhydre.

La préparation, par la voie ainsi définie, de l'hexafluoroacétone comme d'ailleurs des composés fonctionnels perfluorés en général à partir des composés perchlorés correspondants, est très difficile à réaliser.

Les difficultés auxquelles se heurte la réalisation des procédés connus sont exposées dans le brevet français n° 2 135 474. "L'état de la technique montre qu'il est difficile d'obtenir à la fois une perfluoration complète des composés perchlorés en un seul passage des réactifs dans la zone de fluoration en phase vapeur, une productivité convenable et une perte minime de matière utile par suite des réactions parasites de décomposition en composés non utilisables".

Il est connu en effet que lorsque la température à laquelle s'effectue la fluoruration en un seul passage des réactifs sur le catalyseur est relativement basse, c'est-à-dire égale ou inférieure à 250°C, le composé perfluoré désiré ne peut être obtenu qu'avec un rendement et une productivité nettement insuffisants.

La réalisation de la fluoruration en de multiples étapes successives et alternées de séparation et de fluoruration des composés non suffisamment fluorés peut augmenter le rendement en produit perfluoré désiré mais la productivité n'en demeure pas moins extrêmement faible.

Lorsque la réaction de fluoruration est effectuée à température élevée, c-est-à-dire à 300°C au moins, les réactions parasites entraînent rapidement une dégradation excessive des produits et ne permettent pas de profiter économiquement de l'augmentation constatée de la productivité.

Selon le procédé décrit dans le brevet français n° 2 135 474 le composé perfluoré visé n'est obtenu en un seul passage des réactifs sur le catalyseur qu'en réalisant la fluoruration dans trois zones de réaction successives et distinctes l'une de l'autre, la température de chacune des deux dernières zones de réaction étant réglée à une valeur supérieure de 10°C au moins à celle de la température de la zone de réaction qui la précède.

Une telle façon de procéder suppose une technologie complexe, délicate et coûteuse.

Le brevet français n° 2 135 473 revendique un procédé adapté à la mise en oeuvre d'un catalyseur particulier constitué de deux composés minéraux non stoechiométriques, l'un de chrome, l'autre de nickel. L'emploi d'un tel catalyseur conduit malheureusement à une dégradation excessive des molécules organiques, correspondant, en mole, à plus de 7 % de l'hexachloroacétone engagée.

Le brevet FR 2 034 585 relatif à l'hydrofluoration d'hydrocarbures halogénés, pouvant éventuellement renfermer un groupe carbonyle, avec du gaz fluorhydrique en excès à des températures comprises entre 140 et 400°C préconise l'emploi d'un catalyseur constitué d'alumine-$\gamma$ ou -$\eta$ contenant du chrome, de l'oxygène et du fluor. Ce catalyseur est activé avec de l'acide fluorhydrique et de l'air à une température qui ne doit pas dépasser 250°C. Les exemples fournis décrivent l'hydrofluoration du dichloro-1,1 éthylène et du fluorure de vinyle.

Selon le brevet DE 1 443 616 qui concerne la fluoruration catalytique d'hydrocarbures chlorés en deux étapes successives, la température de la seconde étape est supérieure de 30 à 50°C à celle (60-140°C) de la première étape.

Le procédé selon l'invention permet d'atteindre une conversion élevée de l'hexachloroacétone, une productivité convenable et une dégradation pratiquement négligeable des molécules organiques en opérant à haute température et en seulement deux étapes de fluoruration.

Il est caractérisé par l'emploi d'un catalyseur constitué d'une alumine gamma imprégnée de sesquioxyde de chrome $Cr_2O_3$ à raison de 1,5 à 4 et de préférence environ 2,5 atomes de chrome par litre d'alumine et activé entre 300 et 400°C par un mélange d'acide fluorhydrique et de trichloro-1,1,2-tri-fluoro-1,2,2-éthane, dans deux étapes de fluoruration en phase gazeuse, la première consistant à faire réagir à une température comprise entre 300°C et 370°C, un mélange d'acide fluorhydrique et un recyclat de chlorofluoroacitones, la seconde consistant à faire réagir à une température comprise entre 280°C et 320°C un mélange gazeux constitué de l'effluent provenant de la première étape de fluoruration et d'hexafluoroacétone frais de telle façon que rapport du nombre de moles d'acide fluorhydrique introduites dans la première étape de fluoruration au nombre de moles d'halogénoacétones introduites dans la seconde soit compris entre 3 et 5, l'hexafluoroacétone produite étant recueillie à partir de l'effluent gazeux issu de cette seconde étape et les autres halogénoacétones étant recyclées à la première étape de fluoruration.

Dans le procédé selon l'invention l'imprégnation de l'alumine est réalisée par des moyens connus, comme la coulée simultanée et séparée dans un réacteur maintenu en rotation, sous vide, et contenant l'alumine, d'une solution aqueuse d'anhydride chromique $CrO_3$ d'une part, de méthanol d'autre part, suivie d'un séchage du produit obtenu, en lit fixe ou en lit fluide, à une température en général voisine de 150°C sous courant de gaz inerte tel que l'azote.

La réduction du chrome hexavalent en chrome trivalent peut être réalisée par un composé réducteur autre que le méthanol, tel que l'éthanol ou l'hydrazine.

L'activation du catalyseur est réalisée par passage d'un mélange d'acide fluorhydrique et de trichloro-1,1,2 trifluoro-1,2,2 éthane sur le catalyseur séché, de préférence d'abord à 300°C ou 350°C puis à 400°C. Le rapport molaire

HF/$C_2Cl_3F_3$ peut varier de 0,5 à 2, mais est de préférence égal à 1 environ.

La pression absolue à laquelle est effectuée la première comme la seconde étape de fluoruration est généralement de 1 bar environ.

Le débit des réactifs gazeux sur le catalyseur, dans la première comme dans la seconde étape est généralement compris entre 150 et 200 Nl./h./kg de catalyseur.

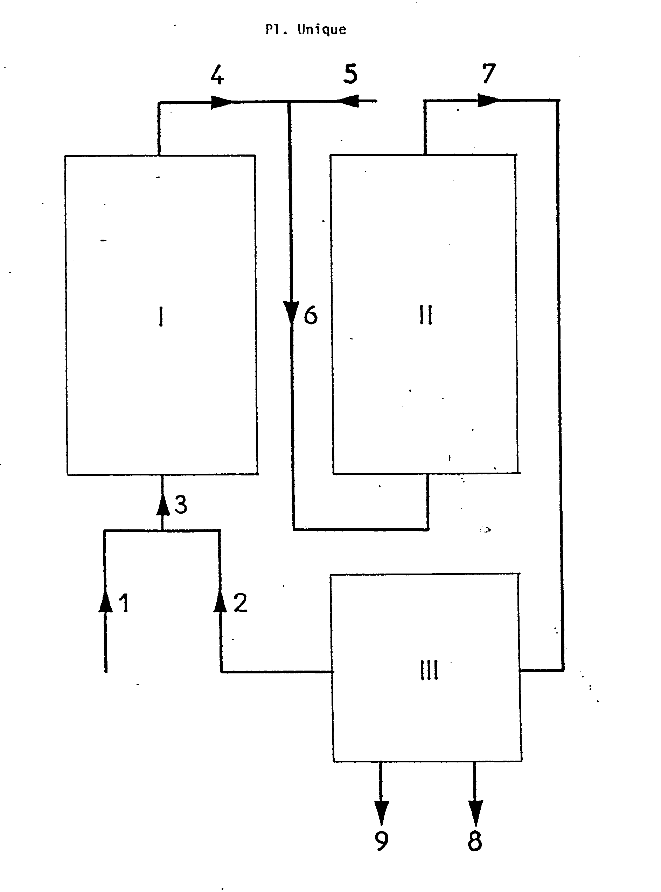

La planche unique est constituée du schéma de réalisation du procédé selon l'invention:

Le mélange constitué d'acide fluorhydrique amené par la tuyauterie 1 et de produits recyclés amenés par la tuyauterie 2 pénètre par la conduite 3 dans le réacteur I qui contient le catalyseur et où s'effectue la première étape de fluoruration en phase gazeuse. L'effluent gazeux issu du réacteur I par la tuyauterie 4 est additionné d'hexachloroacétone gazeux frais amené par la conduite 5 et le mélange ainsi constitué pénêtre par la tuyauterie 6 dans le réacteur II dans lequel s'effectue la seconde étape de fluoruration. Ce second réacteur contient le même catalyseur que celui disposé dans le premier.

L'effluent gazeux issu du réacteur II par la conduite 7 est traité d'une manière connue dans une unité de séparation III d'où est issue, par la tuyauterie 8, l'hexafluoroacétone et, par la conduite 2, les produits recyclés à la première étape de fluoruration. L'acide fluorhydrique non transformé sortant par la conduite 9 est avantageusement recyclé au procédé.

L'exemple suivant, donné à titre non limitatif, illustre le procédé selon l'invention.

## EXEMPLE

Un mélange gazeux constitué d'acide fluorhydrique anhydre et d'un recyclat contenant des chlorofluoroacétones dans la proportion molaire de 1 mole d'acide fluorhydrique pour 0,13 mole de chlorofluoroacétones, est introduit à raison de 173 Nl/h/kg d'un catalyseur disposé dans un premier réacteur de fluoruration de 40 mm de diamètre, et opérant à 365°C. Le catalyseur est préparé et activé avant emploi comme suit: dans un réacteur sphérique maintenu en rotation et sous vide, contenant 0,4 l d'alumine commercialisée par la Société HARSHAW sous la référence Al 111 73 E dont les principales caractéristiques sont:
- présentation : extrudés de 0,8 mm de diamètre environ,
- teneur en $SiO_2$ : 0,13 %
- surface spécifique totale : 161 m2/g
- rayon moyen de pore : 105,g A
- volume total de pore : 0,91 ml/g
- pourcentage de pores compris entre 150 et 250 Å : 16,4
- pourcentage de pores compris entre 250 et 300 Å : 1,7,
sont coulées simultanément et séparement, en 1 heure environ, d'une part 190 g d'une solution aqueuse contenant de l'anhydride chromique $CrO_3$ à une concentration en poids de 52,6 %, d'autre part un mélange de méthanol et d'eau contenant 80 % en poids de méthanol, l'alumine ainsi imprégnée étant ensuite séchée en lit fluide à 150°C sous un courant d'azote.

Une quantité égale à 0,18g kg de catalyseur sec est activée, dans le réacteur de fluoruration, d'abord à 300°C durant 6 heures, puis à 400°C durant 24 heures, par passage de 13,5 Nl/h d'un mélange gazeux équimoléculaire d'acide fluorhydrique et de trichloro-1,1,2-trifluoro-1,2,2-éthane.

A l'effluent gazeux issu du premier réacteur sont ajoutés 3,8 N-l/h d'hexachloroacétone frais gazeux pour former un mélange qui est introduit dans le second réacteur de fluoruration opérant à 300°C. Ce mélange est tel que le rapport du nombre de moles d'acide fluorhydrique introduites dans le premier réacteur au nombre de moles d'halogénoacétones qu'il renferme est égal à 3,8.

Le second réacteur de fluoruration est identique au premier et renferme une quantité égale du même catalyseur activé que ce premier réacteur. Le débit de passage des gaz dans le deuxième réacteur est égal à 193 Nl/h/kg de catalyseur.

L'effluent gazeux issu du second réacteur de fluoruration est traité d'une manière connue pour séparer l'hexafluoroacétone produite, les chlorofluoroacétones en vue de leur recyclage dans le premier réacteur, et l'acide fluorhydrique n'ayant pas réagi en vue de son recyclage avantageux dans ce même réacteur.

Le taux de conversion de l'hexachloroacétone est pratiquement quantitatif, la productivité atteint 46,5 g d'hexafluoroacétone/h/kg de catalyseur, et le taux de décomposition des molécules organiques équivaut à 0,5 % de l'hexachloroacétone engagée.

## Revendications

1. Procédé catalytique continu de préparation d'hexafluoroacétone par fluoruration de l'hexachloroacétone en phase gazeuse par l'acide fluorhydrique anhydre caractérisé en ce qu'un catalyseur constitué d'une alumine gamma imprégnée de sesquioxyde de chrome $Cr_2O_3$ à raison de 1,5 à 4 atomes de chrome par litre d'alumine et activé entre 300°c et 400°C au moyen d'un mélange d'acide fluorhydrique et de trichloro-1,1,2-trifluoro-1,2,2-éthane est mis en oeuvre dans deux étapes de fluoruration, la première consistant à faire réagir à une température comprise entre 330°C et 370°C un mélange d'acide fluorhydrique et de produits recyclés comprenant des chlorofluoroacétones, la seconde consistant à faire réagir à une température comprise entre 280°C et 320°C un mélange constituè de l'effluent gazeux issu de la première étape de fluoruration et d'hexachloroacétone frais de telle façon que le rapport du nombre de moles d'acide fluorhydrique introduites dans la première étape de fluoruration au nombre de moles d'halogénoacétones introduites dans la seconde soit compris entre 3 et 5, l'hexafluoroacétone produite étant recueillie à partir de l'effluent gazeux issu de la seconde étape de fluoruration et les autres halogènoacétones étant recyclées à la première étape de fluoruration.

2. Procédé selon la revendication 1 caractérisé en ce que le débit des réactifs lors de la première étape de fluoruration comme lors de la seconde est compris entre 150 et 200 litres par heure et par kilogramme de catalyseur.

3. Procédé selon la revendication 1 ou 2, dans lequel le catalyseur est activé par un mélange contenant 0,5 à 2 moles, de préférence environ 1 mole, d'acide fluorhydrique par mole de trichloro-1,1,2 tri-fluoro-1,2,2 éthane.

4. Procédé selon l'une dea revendications 1 à 3, dans lequel le catalyseur contient environ 2,5 atomes de chrome par litre d'alumine.

## Patentansprüche

1. Kontinuierliches katalytisches Verfahren zur Herstellung von Hexafluoraceton durch Fluorierung von Hexachloraceton mit wasserfreier Fluorwasserstoffsäure in der Gasphase, dadurch gekennzeichnet, daß in zwei Fluorierungsstufen ein Katalysator aus einem Gamma-Aluminiumoxid eingesetzt wird, der mit Chromsequioxid $Cr_2O_3$ in einer Menge von 1,5 bis 4 Chromatomen je Liter Aluminiumoxid imprägniert und zwischen 300° C und 400° C mit einer Mischung von Fluorwasserstoffsäure und 1,1,2-Trichlor-1,2,2-trifluorethan aktiviert ist, wobei die erste Stufe darin besteht, daß man bei einer Temperatur zwischen 330 und 370° C eine Mischung von Fluorwasserstoffsäure und rückgeführten Produkten, die Chlorfluoracetone enthalten, reagieren läßt, und wobei die zweite darin besteht, daß man bei einer Temperatur zwischen 280° C und 320° C eine Mischung reagieren läßt, die aus dem Austrittsgasstrom aus der ersten Fluorierungsstufe und frischem Hexachloraceton in der Weise besteht, daß das Verhältnis der Molzahlen des in die erste Fluorierungsstufe eingesetzten Fluorwasserstoffs zur Molzahl des in die zweite Stufe eingesetzten Halogenacetons zwischen 3 und 5 liegt, wobei das Hexafluoraceton ausgehend von dem aus der zweiten Fluorierungsstufe austretenden Gasstrom erhalten wird und die anderen Halogenacetone in die erste Fluorierungsstufe rückgeführt werden.

2. Verfahren nach Anspruch 1, dadurch gekennzeichnet, daß die Einsatzmenge der Reaktionspartner bei der ersten Fluorierungsstufe wie bei der zweiten Fluorierungsstufe zwischen 150 und 200 Liter Pro Stunde und pro Kilogramm Katalysator liegt.

3. Verfahren nach Anspruch 1 oder 2, bei dem der Katalysator mit einem Gemisch aktiviert wird, das 0,5 bis 2 Mol, vorzugsweise 1 Mol Fluorwasserstoffsäure pro Mol 1,1,2-Trichlor-1,2,2-trifluor-ethan enthält.

4. Verfahren nach einem der Ansprüche 1 bis 3, bei dem der Katalysator etwa 2,5 Atome Chrom Pro Liter Aluminiumoxid enthält.

## Claims

1. Continuous catalytic process for the preparation of hexafluoroacetone by gas phase fluorination of hexachloroacetone by means of anhydrous hydrofluoric acid, characterized in that a catalyst consisting of a gamma alumina impregnated with chromium sesquioxide $Cr_2O_3$ in a proportion of 1.5 to 4 chromium atoms per litre of alumina and activated between 300° C and 400° C by means of a mixture of hydrofluoric acid and 1,1,2-trichloro-1,2,2-trifluoroethane is used in two fluorination stages, the first consisting in reacting, at a temperature of between 330° C and 370° C, a mixture of hydrofluoric acid and of recycled products comprising chlorofluoroacetones, the second consisting in reacting, at a temperature of between 280° C and 320° C, a mixture consisting of the gaseous effluent originating from the first fluorination stage and of fresh hexachloroacetone so that the ratio of the number of moles of hydrofluoric acid introduced in the first fluorination stage to the number of moles of haloacetones introduced in the second stage is between 3 and 5, the hexafluoroacetone produced being collected from the gaseous effluent originating from the second fluorination stage and the other haloacetones being recycled to the first fluorination stage.

2. Process according to Claim 1, characterized in that the flow rate of the reactants during the first fluorination stage, as during the second stage, is between 150 and 200 litres per hour and per kilogram of catalyst.

3. Process according to Claim 1 or 2, in which the catalyst is activated with a mixture containing 0.5 to 2 moles, preferably approximately 1 mole, of hydrofluoric acid per mole of 1,1,2-trichloro-1,2,2-trifluoroethane.

4. Process according to one of Claims 1 to 3, in which the catalyst contains approximately 2.5 chromium atoms per litre of alumina.

Pl. Unique